(19) Europäisches Patentamt
European Patent Office
Office européen des brevets

(11) Numéro de publication: 0 004 407 B2

(12) # NOUVEAU FASCICULE DE BREVET EUROPEEN

(45) Date de publication du nouveau fascicule du brevet: **30.03.88**

(51) Int. Cl.⁴: **C 07 C 179/10**, C 07 D 301/14

(21) Numéro de dépôt: **79200137.2**

(22) Date de dépôt: **21.03.79**

(54) **Procédé pour la fabrication de peracides carboxyliques et utilisation ceux ci pour la fabrication d'époxydes à partir d'oléfines.**

(30) Priorité: **28.03.78 FR 7809199**

(43) Date de publication de la demande:
**03.10.79 Bulletin 79/20**

(45) Mention de la délivrance du brevet:
**12.08.81 Bulletin 81/32**

(45) Mention de la décision concernant l'opposition:
**30.03.88 Bulletin 88/13**

(84) Etats contractants désignés:
**BE CH DE GB IT LU NL SE**

(56) Documents cités:
**BE - A - 847 664**
**DE - A - 2 519 297**
**DE - B - 1 152 415**
**DE - B - 1 184 343**
**FR - A - 1 252 694**
**FR - A - 2 300 085**
**US - A - 2 814 641**
**US - A - 2 903 465**

**D. Swern - Organic Peroxides, volume II, Wiley Interscience, 1971, p. 355-533**
**Kirk-Othmer - Encyklopedia of Chemical Technology, volume 8, 2nd Edn, 1965, p. 238-261**
**Kirk-Othmer - Encyklopedia of Chemical Technology, volume 9, 3rd Edn, 1980, pages 252, 254, 259, 260.**
**J. of Org. Chem., 1958, 23, pages 1823 à 1826**

(73) Titulaire: **PROPYLOX (Société Anonyme), 12, avenue de la Renaissance, B-1040 Bruxelles (BE)**

(72) Inventeur: **Hardy, Nicolas, Rue de la Taille, 52, B-5790 Jemeppe-sur-Sambre (BE)**
Inventeur: **Lerot, Luc, Rue de l'équerre 15, B-1140 Bruxelles (BE)**
Inventeur: **Walraevens, René, Avenue des neuf Provinces, 3, B-1080 Bruxelles (BE)**

(74) Mandataire: **Lederer, Franz, Dr., Van der Werth, Lederer & Riederer Patentanwälte Lucile-Grahn-Strasse 22, D-8000 München 80 (DE)**

EP 0 004 407 B2

ACTORUM AG

## Description

La présente invention concerne un procédé pour la fabrication de peracides carboxyliques en continu par réaction des acides carboxyliques correspondants avec le peroxyde d'hydrogène en présence d'un catalyseur. Elle concerne également l'utilisation des peracides obtenus pour la fabrication d'époxydes à partir d'oléfines.

Dans la demande de brevet français 2 300 085 déposée le 2 février 1976 au nom d'Interox Chemicals Ltd, on propose un procédé pour la fabrication de peracides carboxyliques qui consiste à faire passer à contre-courant, dans un réacteur-extracteur, une solution aqueuse contenant de l'acide sulfurique et du peroxyde d'hydrogène et une solution organique d'acide carboxylique. Aux sorties du réacteur, on recueille une solution organique d'acide percarboxylique et une solution aqueuse diluée en acide sulfurique, qu'il est nécessaire de concentrer avant de la recycler au réacteur.

Il est également connu de fabriquer des peracides carboxyliques en faisant réagir l'acide carboxylique correspodant avec du peroxyde d'hydrogène mis en œuvre généralament sous forme d'une solution aqueuse en présence de petites quantités d'un catalyseur tel que l'acide sulfurique. Cette réaction donne lieu à la formation d'eau. Pour obtenir le peracide carboxylique directement sous forme anhydre, on a proposé dans le brevet Etats-Unis 2 814 641, déposé le 31 juillet 1956 et cédé à Union Carbide Corporation, d'opérer la réaction en présence d'un solvant capable de former un azéotrope à minimum avec l'eau et d'éliminer l'eau formée par la réaction ainsi que l'eau de dilution des réactifs par distillation de cet azéotrope.

Ce procédé connu présente certains inconvénients graves. En effet, la proportion de composés peroxydés (peroxyde d'hydrogène et peracide carboxylique) présente dans le mélange réactionnel est très important et va en s'accroissant au fur et à mesure de l'avancement de la réaction et de la distillation azéotropique. Ceci entraîne des risques d'explosion qui rendent la réaction particulièrement difficile à conduire. D'autre part, dans ce procédé connu, en fin de processus, le catalyseur se retrouve dans la solution organique de peracide et il est dès lors nécessaire de prévoir l'élimination de ce catalyseur. Cette élimination est extrêmement difficile à réaliser. Par ailleurs la présence de catalyseur résiduaire dans la solution organique de peracide se révèle très gênante pour tous les usages ultérieurs de cette solution tels que l'emploi comme agent d'époxydation. En outre ce procédé conduit à une consommation importante en catalyseur que l'on ne peut pas récupérer. De ce fait, pour limiter les inconvénients liés à la présence du catalyseur dans la solution organique de peracide on est amené à utiliser de faibles quantités relatives de catalyseur ne dépassant en général pas 5% du poids d'acide carboxylique mis en œuvre, ce qui a pour conséquence néfaste de réduire sensiblement les vitesses de réaction. Enfin comme la réaction de formation du peracide a lieu principalement dans la phase aqueuse et que par ailleurs cette phase aqueuse est éliminée par distillation azéotropique la vitesse de production du peracide décroît très sensiblement au cours du temps au fur et à mesure de la disparition de la phase aqueuse. Pour atteindre des taux de transformation élevés il y a donc lieu d'utiliser des temps de réaction très longs. De ce fait, ce procédé connu ne peut que très difficilement être mis en œuvre dans des installations fonctionnant en continu.

On a maintenant trouvé un procédé qu'il est très facile de mettre en œuvre en continu et qui ne présente pas les inconvénients cités ci-dessus.

La présente invention concerne donc un procédé pour la fabrication de peracides carboxyliques en continu par réaction de l'acide carboxylique correspondant avec le peroxyde d'hydrogène en présence d'un catalyseur et d'un liquide organique inerte, solvant du peracide et capable de former avec l'eau un azéotrope hétérogène, dans lequel on élimine de l'eau présente dans le mélange réactionnel par distillation de l'azéotrope eau-liquide organique, et dans lequel on maintient dans le mélange réactionnel une quantité d'eau suffisante pour qu'il se forme une phase aqueuse distincte d'une phase organique qui contient de l'acide carboxylique, du peracide carboxylique et le liquide organique et dans lequel on prélève une partie du mélange réactionnel et on sépare la phase aqueuse de la phase organique.

En général, on maintient dans le mélange réactionnel une quantité d'eau suffisante pour que, dans le mélange réactionnel, le rapport pondéral de la phase aqueuse par rapport à la phase organique soit supérieur à 0,05. De préférence, ce rapport est supérieur à 0,1. Les meilleurs résultats sont obtenus lorsque ce rapport est supérieur à 0,2.

Par ailleurs, il n'y a pas intérêt, dans la plupart des cas, à maintenir dans le mélange réactionnel des quantités d'eau telles que le rapport pondéral de la phase aqueuse par rapport à la phase organique soit supérieur à 20. De préférence, ce rapport est inférieur à 10. Les meilleurs résultats sont obtenus lorsqu'il est inférieur à 5.

La phase aqueuse comprend en général de 5 à 95% et le plus souvent de 10 à 70% de son poids d'eau, le solde étant substantiellement formé par les constituants du mélange réactionnel et principalement par le peroxyde d'hydrogène et le catalyseur lorsque ce dernier est soluble et ne se présente pas sous forme de suspension solide. Elle comprend également, en général, une partie de l'acide et du peracide carboxyliques.

L'eau présente dans la phase aqueuse peut provenir notamment de la réaction ou de l'introduction de certains constituant de mélange réactionnel, en général le peroxyde d'hydrogène, et éventuellement le catalyseur, sous forme de solutions aqueuses. Elle peut aussi avoir été ajoutée intentionnellement.

La phase organique comprend en général de 30 à 98% et le plus souvent de 40 à 95% de son poids de liquide organique, le solde étant substantiellement formé par des constituants du mélange réactionnel et principalement par l'acide carboxylique et le peracide carboxylique.

Elle peut également contenir de petites quantités de peroxyde d'hydrogène et éventuellement de catalyseur. En général la teneur en peroxyde d'hydrogène dans la phase organique ne dépasse pas 5% de son poids et sa teneur en catalyseur ne dépasse pas 1% de son poids. Le plus souvent les teneurs en peroxyde d'hydrogène et en catalyseur de la phase organique ne dépassent pas respectivement 2 et 0,4% de son poids.

Le liquide organique mis en œuvre dans le mélange réactionnel doit être inerte vis-à-vis des divers constituants du mélange réactionnel dans les conditions de la réaction. En outre, il doit pouvoir former avec l'eau un azéotrope hétérogène à minimum dont la température d'ébullition doit être inférieure, dans des conditions de pressions égales, à la température d'ébullition des autres constituants et des autres azéotropes éventuels qui pourraient être formés dans le mélange réactionnel.

Enfin, il doit dissoudre le peracide carboxylique formé au cours de la réaction, et de préférence suffisamment pour que, dans les conditions de réaction, la concentration, exprimée en mole par litre du peracide dans la phase organique soit égale à au moins 0,05 et de préférence au moins 0,2 fois la concentration du peracide dans la phase aqueuse.

Selon un mode de réalisation préféré du procédé selon l'invention, on prélève en continu une partie du mélange réactionnel et on sépare par décantation la phase aqueuse de la phase organique dans la partie prélevée. De préférence, la phase aqueuse ainsi séparée est réintroduite dans le mélange réactionnel. La phase organique ainsi séparée constitue la production.

Ce mode de réalisation est particulièrement avantageux lorsqu'on met en œuvre un liquide organique très peu soluble dans l'eau et dans lequel l'eau est peu soluble. De préférence on choisit un liquide organique tel que la teneur en eau de la phase organique est inférieure à la teneur en eau de l'azéotrope eau-liquide organique dans les mêmes conditions de température et de pression; le plus souvent il est choisi de manière à ce que la quantité d'eau dans la phase organique soit inférieure à 5% et de préférence inférieure à 1%. Par contre la quantité de liquide organique dissous dans la phase aqueuse est moins critique; en général on veille à choisir un liquide organique tel qu'elle ne dépasse pas 10% et le plus souvent 5%. En outre on choisit un liquide organique tel que les densités des phases aqueuses et organiques soient suffisamment différentes pour en permettre la décantation.

L'invention concerne également l'utilisation de la phase organique obtenue à la décantation selon le mode de réalisation précitée, sans séparation préalable en ses constituants principaux, pour la fabrication d'époxydes à partir d'oléfines, selon des procédés qui sont bien connus en eux-mêmes.

Elle contient des quantités variables de peracide de carboxylique, qui sont en général comprises entre 5 et 40% en poids. Avant d'être mise en œuvre à l'époxydation elle peut être soumise à divers traitements, par exemple pour éliminer les dernières traces d'humidité et de catalyseur. Ceux-ci ne sont cependant pas indispensables. Lors de la réaction d'époxydation, le rapport molaire entre le peracide carboxylique et l'oléfine à époxyder est en général compris entre 0,01 et 20. Il est de préférence compris entre 0,1 et 10. On peut également ajouter au mélange réactionnel de petites quantités d'additifs divers tels que des inhibiteurs de polymérisation, des stabilisants du peracide, ou des séquestrants.

La réaction d'époxydation est réalisée en général à des températures comprises entre 0 et 150°C. Celles-ci sont, de préférence, comprises entre 15 et 120°C. La pression de réaction est en général suffisante pour maintenir au moins une phase liquide. Elle est en général comprise entre 0,05 et 80 kg/cm². Bien entendu la température et la pression de réaction dépendent de la nature particulière de l'oléfine à époxyder. Ainsi pour époxyder le propylène on utilise le plus souvent une température de 20 à 100°C et une pression de 0,8 à 30 kg/cm². Pour époxyder le chlorure d'allyle et l'alcool allylique, on utilise le plus souvent une température de 20 à 150°C et une pression de 0,1 à 10 kg/cm². Les réacteurs utilisés pour effectuer la réaction d'époxydation sont en général des réacteurs favorisant les échanges thermiques de manière à mieux contrôler les températures de réaction. On peut ainsi utiliser des réacteurs tubulaires ou des autoclaves, un réacteur unique ou des réacteurs en cascades.

Le mélange réactionnel obtenu à l'époxydation est constitué essentiellement du liquide organique, d'oxyde d'oléfine, d'acide carboxylique et de réactifs non transformés; il peut éventuellement contenir de petites quantités de sous-produits et d'additifs divers. Il est, habituellement, soumis à une première séparation de manière à récupérer l'oléfine non transformée d'une part et une première solution organique liquide constituée essentiellement de liquide organique, de l'oxyde d'oléfine, d'acide carboxylique et éventuellement de peracide carboxylique non transformé.

Dans le cas d'olefines légères, le propylène par exemple, cette séparation se fait avantageusement par simple détente rapide à la pression atmosphérique. Pour les oléfines plus lourdes, le chlorure d'allyle ou l'alcool allylique par exemple, on peut effectuer cette première séparation par distillation. L'oléfine recueillie est avantageusement recyclée à la réaction d'époxydation. Pour ce faire, on peut absorber l'oléfine sous forme gazeuse dans la phase organique contenant le peracide avant de l'envoyer à l'époxydation. On

peut également condenser l'oléfine et ensuite seulement l'envoyer à l'époxydation.

La première solution organique liquide recueillie à la première séparation est habituellement soumise à une seconde séparation, avantageusement par distillation, de façon à récupérer l'oxyde d'oléfine souhaité, d'une part, et une seconde solution organique d'acide carboxylique dans le liquide organique, d'autre part. L'oxyde d'oléfine peut être utilisé tel quel ou être soumis à certaines étapes d'épuration ultérieures pour en éliminer les traces éventuelles de sous-produits tels que les aldéhydes.

Selon la variante présentement décrite, la solution d'acide carboxylique dans le liquide organique, qui contient en outre, éventuellement, le peracide carboxylique non transformé ainsi que certains sous-produits et additifs tels que ceux cités ci-avant est renvoyée directement à la fabrication du peracide. Avantageusement, cette solution est préchauffée avant d'être introduite dans la zone de réaction de manière à apporter au moins une partie de la chaleur nécessaire à la distillation azéotropique.

Lorsqu'on utilise cette variante préférée, le liquide organique est choisi parmi ceux dont la température d'ébullition est supérieure à celles de l'oléfine et de l'oxyde d'oléfine. Par ailleurs, il convient que le liquide organique ne forme pas d'azéotrope avec l'oléfine et l'oxyde d'oléfine. Enfin dans le cas où il est capable de former un azéotrope avec l'acide carboxylique ou le peracide carboxylique, il convient que les températures d'ébullition de ces azéotropes soient supérieures à celles de l'oléfine et de l'oxyde d'oléfine.

Tous les composés organiques liquides dans les conditions de réaction répondant aux conditions définies ci-avant peuvent convenir pour réaliser le procédé selon l'invention. Ces liquides sont en général choisis parmi les esters carboxyliques, les éthers, les hydrocarbures halogénés, les hydrocarbures non substitués, les hydrocarbures substitués par des groupes nitro, les esters non acides d'acides nitrique, carbonique et phosphorique, et leurs mélanges.

Comme esters carboxyliques convenant bien en général, on peut signaler les esters aliphatiques, alicycliques ou aromatiques d'acides mono- ou poly-carboxyliques avec des alcools mono- ou poly-hydriques contenant de 4 à 20 et de préférence de 4 à 10 atomes de carbone dans la molécule. Parmi ceux-ci, conviennent particulièrement bien les formiates et acétates d'isopropyle, de propyle, de butyle, d'isobutyle, de secbutyle, de tert-butyle, d'amyle, d'isoamyle et de secamyle, les mono- et dichloroacétates, propionates, butyrates et isobutyrates de méthyle, d'éthyle, de propyle d'isopropyle, de butyle, d'isobutyle et d'isoamyle, les valérates, l'isovalérates et caproates de méthyle, d'éthyle et de propyle, les acétates de méthoxyéthyle, d'éthoxyéthyle et de cyclohexyle, le pivalate de méthyle et les esters diéthyliques des acides phtalique et adipique.

Comme éthers convenant bien en général, on peut signaler les éthers aliphatiques symétriques ou asymétriques contenant de 4 à 12 atomes de carbone tels que le 2,2'-dichlorodiéthyl éther, le butyléthyl éther, le tert-butyléthyl éther, le tert-amylméthyl éther, le diisopropyl éther, le dipropyl éther, le dibutyl éther, l'éthylhexyl éther et le diisobutyl ether.

Comme hydrocarbures halogénés convenant bien en général, on peut signaler les hydrocarbures halogénés aromatiques, aliphatiques et alicycliques contenant de 1 à 8 atomes de carbone dans leur molécule substitués par au moins un halogène choisi de préférence parmi le chlore, le fluor et le brome. Des hydrocarbures halogénés convenant particulièrement bien sont le tétrachlorure de carbone, le chloroforme, le chlorure de méthylène, les di-, les tri-, les tétra- et le pentachloréthanes, les trichlorotrifluoréthanes, le tri- et le tétra-chloréthylène, les mono-, les di- et les tri-chloropropanes, les butanes, méthylpropanes, pentane et hexanes mono- ou poly-chlorés, le mono- et les dichlorobenzènes, et les chlorotoluènes.

Comme hydrocarbures substitués par des groupes nitro convenant bien en général, on peut mentionner les hydrocarbures contenant de 3 à 8 atomes de carbone aromatiques, aliphatiques, ou alicycliques, tels que les nitro-propanes, le nitrobenzène et le nitrocyclohexane.

Comme hydrocarbures non substitués convenant bien en général, on peut signaler les hydrocarbures contenant de 5 à 14 atomes de carbone aliphatiques, aromatiques, ou alicycliques, tels que le benzène, le toluène, le xylène, le pentane, l'hexane, l'heptane, l'octane, le diisobutyle, le cyclohexane, le méthylcyclohexane et la tétraline.

Comme esters d'acide carbonique convenant bien en général, on peut mentionner les esters aliphatiques contenant de 3 à 9 atomes de carbone dans la molécule tels que les carbonates de diméthyle, diéthyle, diisobutyle, dibutyle, di-tert-butyle, dipropyle et diisopropyle. Des esters d'acide nitrique convenant bien en général sont ceux choisis parmi les esters aliphatiques contenant de 1 à 5 atomes de carbone dans la molécule tels que les nitrates de méthyle, propyle, butyle et isoamyle. Quant aux esters d'acide phosphorique convenant bien, ce cont ceux qui répondent à la formule

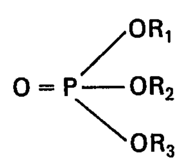

où $R_1$, $R_2$, et $R_3$ sont identiques ou différents et représentent des groupes alkyles, aryles, arylalkyles ou alkylaryles tels que la molécule contient de 3 à 30 atomes de carbone. A titre d'exemples de phosphates particuliers, on peut signaler les phosphates de triméthyle, de tributyle, de trioctyle et de dioctylphényle.

Des liquides organiques qui sont particulièrement adéquats pour être utilisés dans la fabrication des peracides acétique et propionique sont

le benzène, le toluène, le 1,2-dichloropropane, le 1,1,2,2-tétrachloréthane, le pentachloréthane, le tétrachloréthylène, le 1-nitropropane, le chlorobenzène, le parachlorotoluène, le chloroacétate de méthyle, le carbonate de diéthyle, le dichloréthane, l'acétate de butyle, le cyclohexane et le phosphate de tributyle. Des résultats particulièrement bons sont obtenus avec le 1,2-dichloropropane, le 1,2-dichloréthane, le 1,1,2,2-tétrachloréthane, et leurs mélanges.

L'azéotrope recueilli par distillation est en général condensé et soumis à une décantation de manière à séparer l'eau du liquide organique. Le liquide organique ainsi recueilli peut être avantageusement utilisé, au moins partiellement, pour assurer le reflux dans la zone de distillation. Il peut aussi être réintroduit, en général après vaporisation, dans la zone de réaction pour servir à former la phase organique. Cette introduction sous forme vapeur permet d'apporter au moins en partie la chaleur nécessaire à la vaporisation de l'azéotrope eau-liquide organique.

Le procédé de l'invention peut être appliqué à la fabrication d'un grand nombre de peracides carboxyliques. Ainsi, il peut être utilisé pour former des peracides au départ d'acides mono- ou polycarboxy-liques. Dans ce dernier cas, l'acide polycarboxylique peut être mis en œuvre dans le procédé selon l'invention également sous la forme de l'anhydride correspondant. Le procédé selon l'invention convient particulièrement bien pour la peracidification d'acides carboxyliques contenant de 1 à 10 atomes de carbone tels que des acides carboxyliques aliphatiques, alicycliques ou aromatiques tels que l'acide formique, l'acide acétique, les acides chloroacétiques, l'acide propionique, l'acide butanoïque, l'acide ou l'anhydride maléique, l'acide benzoïque, l'acide cyclohexanecarboxylique et les acides et anhydride phtalique. Des résultats particulièrement avantageux sont obtenus lors de la fabrication des peracides acétiques et propioniques au départ des acides acétiques et propioniques respectivement.

Le catalyseur mis en œuvre est en général un catalyseur acide convenant pour les réactions d'estérification tel que par exemple l'acide sulfurique, les acides alkyl-, aryl-, arylalkyl-, alkylarylsulfoniques, l'acide phosphorique, les phosphates acides d'alkyle, d'aryle, d'alkylaryle et d'arylalkyle, l'acide trifluoracétique, l'acide acétylsulfoacétique ainsi que des résines échangeuses d'ions du type polymères ou copolymères sulfonés. Comme catalyseurs préférés, on peut signaler plus particulièrement l'acide sulfurique et les acides méthane-, éthane-, benzène-, toluène-, xylène-, butane-, propane-, et napthalène-sulfoniques. Parmi ces catalyseurs, on préfère utiliser ceux qui sont solubles dans l'eau et ne sont pas, ou peu, solubles dans le liquide organique. Les meilleurs résultats sont obtenus avec les catalyseurs solubles dans l'eau dont la concentration dans la phase organique est, dans les conditions de réaction, inférieure à 3% et de préférence inférieure à 1% en poids. Des résultats particulièrement avantageux ont été obtenus avec l'acide sulfurique.

La concentration en catalyseur dans le mélange réactionnel peut varier dans de larges proportions. Pour obtenir des vitesses réactionnelles élevées, on utilise en général des concentrations en catalyseurs importantes. En général, on utilise une quantité de catalyseur supérieure à 5% du poids total d'acide et de peracide carboxyliques présents dans le mélange réactionnel. La teneur pondérale en catalyseur est de préférence comprise entre 0,1 et 30 fois le poids total d'acide et de peracide carboxyliques présents dans le mélange réactionnel. Les meilleurs résultats sont obtenus lorsque cette teneur est comprise entre 0,2 et 10 fois le poids total d'acide et de peracide carboxyliques.

Le catalyseur peut être mis en œuvre à l'état pur. Cependant, il est avantageux de le mettre en œuvre sous forme d'une solution aqueuse s'il est soluble dans l'eau. Dans ce cas, on peut avantageusement mettre le catalyseur en œuvre en réintroduisant dans le mélange réactionnel la phase aqueuse provenant de la décantation de celui-ci, au besoin après avoir ajouté un appoint de catalyseur. En général, la concentration en catalyseur soluble dans l'eau dans la phase aqueuse est comprise entre 10 et 60% en poids.

L'acide carboxylique peut être mis en œuvre dans le procédé selon l'invention à l'état pur. En général, on le met en œuvre cependant sous la forme d'une solution dans le liquide organique. Pareilles solutions contenant de 2 à 70%, et de préférence de 5 à 60%, en poids d'acide carboxylique sont avantageusement introduites dans le mélange réactionnel. Pour préparer ces solutions, on peut utiliser du liquide organique provenant de la décantation de l'azéotrope distillé, du liquide organique frais, ou encore du liquide organique récupéré après utilisation de la solution organique de peracide.

Le peroxyde d'hydrogène utilisé pour la réaction peut être mis en œuvre soit à l'état pur soit à l'état de solutions acqueuses.

Le peroxyde d'hydrogène peut être mis en œuvre sous la forme d'une solution aqueuse. Avantageusement, on utilise des solutions concentrées en peroxyde d'hydrogène contenant de 20 à 90% en poids de peroxyde d'hydrogène. D'autres concentrations peuvent également convenir mais sont moins favorables. En effet, aux concentrations plus faibles en peroxyde d'hydrogène, les quantités d'eau à éliminer par distillation azéotropique sont très importantes tandis que des solutions plus fortement concentrées en peroxyde d'hydrogène sont difficiles à produire industriellement.

Dans le mélange réactionnel, les proportions de réactifs peuvent varier, dans l'absolu et l'une par rapport à l'autre, à l'intérieur de larges limites en fonction notamment des vitesses d'introduction choisies pour les réactifs. Ainsi, la quantité de peroxyde d'hydrogène est en général comprise entre 0,1 et 10, et de préférence entre 0,2 et 5 mole par mole de fonction carboxylique. Les résultats les plus avantageux sont obtenus d'habitude lorsqu'on introduit dans le mélange réactionnel des quantités de peroxyde d'hydrogène et d'acide carboxylique dans un rapport voisin de la stoechiométrie ou légèrement inférieur. On introduit donc de préférence le peroxyde d'hydrogène et l'acide carboxylique en quantités telles que l'on introduit entre 0,2 et 2 et de préférence entre 0,4 et 1,2 mole de peroxyde d'hydrogène par mole de fonction carboxylique.

Le peroxyde d'hydrogène peut être introduit directement dans le réacteur ou dans la solution aqueuse de catalyseur envoyée au réacteur, quand ce dernier est soluble dans l'eau. Avantageusement on introduit le peroxyde d'hydrogène dans la solution aqueuse de catalyseur envoyée au réacteur. Le plus souvent le peroxyde d'hydrogène est introduit dans la phase aqueuse recueillie par décantation du mélange réactionnel et recyclée en navette au réacteur. Cette introduction se fait avantageusement de façon étagée de manière à éviter des concentrations locales trop élevées en peroxyde d'hydrogène. Le débit de la navette de phase aqueuse doit être suffisant pour que la composition de la phase aqueuse enrichie en peroxyde d'hydrogène obtenue soit toujours telle que le mélange réactionnel reste en dehors des limites d'explosibilité.

La température du mélange réactionnel est choisie en général inférieure à 100°C et, le plus souvent, est comprise entre 20 et 70°C. Des températures supérieures sont moins intéressantes car elles entraînent un risque de décomposition brutale des composés peroxydés. La pression est réglée en fonction de la température de manière à maintenir l'ébullition. Elle peut de ce fait varier dans de larges proportions. Elle est le plus souvent comprise entre 0,01 et 1,2 kg/cm$^2$.

La chaleur nécessaire pour maintenir l'ébullition peut être apportée selon des techniques classiques connues en elles-mêmes. On peut ainsi réchauffer le mélange réactionnel (phase aqueuse et phase organique) par mise en contact avec une surface d'échange chauffée au moyen d'un fluide caloporteur tel que la vapeur d'eau. On peut également, de façon avantageuse, introduire sous forme de vapeur le liquide organique, l'acide carboxylique, ou encore leurs mélanges dans le mélange réactionnel.

Pour réaliser le procédé selon l'invention, on peut utiliser n'importe quel appareillage convenant pour les mélanges réactionnels liquides et notamment les réacteurs à cuve dotés d'un système d'agitation. Plus particulièrement, il est avantageux d'utiliser des réacteurs, connus en eux-mêmes, permettant la distillation en cours de réaction d'un des constituants d'un mélange réactionnel liquide. En général, il s'agit de réacteurs permettant d'assurer un mélange intime des phases aqueuses et organique et un bon échange entre les phases liquides et la phase gazeuse de manière à favoriser la vaporisation de l'azéotrope eau-liquide organique.

Ces réacteurs sont avantageusement couplés à des colonnes de distillation, connues en elles-mêmes, telles que des colonnes à plateau ou à empilage.

Les diverses parties des réacteurs et des colonnes en contact avec le mélange réactionnel sont avantageusement réalisées dans des matériaux résistants à la corrosion tels que les aciers inoxydables, les alliages connus sous les marques INCONEL, HASTELLOY, INCOLOY, NIMONIC, NI-RESIST, et CHLORIMET, et les aciers émaillés.

La séparation par décantation du mélange réactionnel soutiré du réacteur et celle de l'azéotrope eau-liquide organique recueilli en tête de colonne peut être réalisée selon diverses techniques connues en elles-mêmes telles que décantation par gravité ou par action de la force centrifuge ou passage au travers de membranes poreuses sélectivement mouillées par l'une ou l'autre phase. Divers types d'appareils connus en eux-mêmes peuvent être utilisés à cette fin. On peut ainsi utiliser des décanteurs florentins, des décanteurs centrifuges, des filtres séparateurs à membranes ou des séparateurs électriques. La séparation par décantation peut être facilitée par une opération préalable de caolescence des gouttelettes dans des appareils connus en eux-mêmes tels que des matelas ou des cartouches en matériaux fibreux mouillables de préférence par la phase dispersée.

Le procédé selon l'invention peut être réalisé en continu dans un appareil tel que celui représenté schématiquement à la figure unique du dessin en annexe qui se rapporte à un mode de réalisation pratique particulier.

Dans un réacteur 1 surmonté d'une colone de distillation 2, on introduit par la voie 23 une solution concentrée de peroxyde d'hydrogène et de catalyseur obtenue par mélange dans le mélangeur 22 de peroxyde d'hydrogène aqueux introduit par la voie 3 avec du catalyseur introduit par la voie 8 et par la voie 5 une solution d'acide carboxylique dans un liquide organique obtenue par mélange dans le mélangeur 6 de l'acide carboxylique introduit par la voie 4 avec le liquide organique introduit par la voie 7.

En cours de réaction, l'azéotrope eaux-liquide organique quitte la colonne de distillation 2 par la voie 9, est condensé dans le condenseur 10, et est envoyé par la voie 11 au décanteur 12. Lorsque le liquide organique a une densité supérieure à celle de l'eau, on recueille en tête du décanteur l'eau par la voie 13 et le liquide organique en pied du décanteur par la voie 14; dans le cas contraire les prélèvements sont inversés. Le liquide organique est recyclé à la colonne de distillation par la voie 15 oú il constitue le reflux. Dans certains cas, on peut envoyer une partie de ce liquide organique par la voie 16 dans le mélangeur 6 où il set de solvant de l'acide carboxylique.

On soutire en continu du réacteur mélangeur par la voie 17 une partie du mélange réactionnel que l'on envoie au décanteur 18. Lorsque la densité de la phase organique est inférieure à celle de la phase aqueuse, on soutire en tête du décanteur 18, (par la voie 20), la phase organique qui

contient le peracide carboxylique produit et en pied du décanteur 18 la phase aqueuse qui est recycliée au réacteur par la voie 19. Une purge 21 permet d'éliminer une partie des sous produits qui s'accumulent dans la phase aqueuse. La phase organique recueillie par la voie 20 peut être directement utilisée telle quelle notamment pour réaliser des réactions d'époxydation, ou encore être soumise à des traitements d'épuration pour en éliminer les dernières traces d'humidité ou de catalyseur.

Le procédé selon l'invention se révèle particulièrement intéressant car il permet l'obtention en continu de solutions organiques sensiblement anhydres et concentrées en peracide carboxylique. En outre, les risques d'explosion dus à la décomposition des composés peroxydés dans le milieu réactionnel y sont fortement réduits étant donné que la concentration totale en composés peroxydés est maintenue à un niveau constant et qu'on n'observe jamais de pointes de concentrations en composés peroxydés. La proportion totale de composés peroxydés dans le mélange réactionnel reste en permanence à un niveau relativement faible. De plus, le taux de transformation des réactifs est excellent. De même, le procédé n'implique pas la destruction du catalyseur et ne nécessite aucun processus compliqué de récupération du catalyseur, et en particulier aucune distillation. Enfin, le procédé autorise le choix de conditions de réaction permettant d'atteindre des vitesses de réaction remarquablement élevées.

Les peracides carboxyliques obtenus selon le procédé de la présente invention peuvent être utilisés comme source d'oxygène actif dans de nombreuses réactions chimiques et plus particulièrement pour la fabrication d'époxydes à partir d'oléfines. A cet effet, on peut mettre en œuvre n'importe quel composé organique, éventuellement substitué, comprenant au moins une liaison carbone-carbone insaturée, et plus particulièrement comprenant de 2 à 20 atomes de carbone dans sa molécule. A titre d'exemples de pareilles oléfines, on peut citer le propylène, le chlorure d'allyle, l'alcool allylique, et le styrène.

Afin d'illustrer l'invention, sans pour autant en limiter la portée, on donne ci-après un exemple de fabrication de peracide carboxylique.

Exemple

L'appareil utilisé est semblable à celui schématisé à la figure 1.

Le réacteur, d'une contenance de 1 l, contient au départ 0,2 kg d'acide sulfurique à 45%.

La température du réacteur est maintenue en vigueur à environ 39°C, la pression y est d'environ 100 mm de mercure.

On introduit dans le réacteur, en continu, 0,12 kg par heure d'une solution aqueuse à 70% en poids de peroxyde d'hydrogène et 1,07 kg par heure d'une solution à 27% en poids d'acide propionique dans le 1,2-dichloropropane.

Le rapport pondéral de la phase aqueuse à la phase organique présentes dans le réacteur est de 1,04. Un système d'agitation maintient la phase aqueuse et la phase organique en émulsion.

On soutire en continu une partie du mélange réactionnel. Après décantation de la fraction soutirée on obtient 1,11 kg par heure d'une solution organique ayant la composition suivante:

| | g/kg |
|---|---|
| acide perpropionique | 200 |
| acide propionique | 92,2 |
| peroxyde d'hydrogène | 2,89 |
| eau | traces |
| Acide sulfurique | 1,27 |
| 1,2-dichloropropane | 703,64 |

L'examen des résultats obtenus à l'exemple montre qu'il est possible en utilisant le procédé de l'invention d'obtenir des solutions organiques fort concentrées en acide perpropionique (20%) quasiment exempte d'eau et de catalyseur.

La solution organique concentrée ainsi obtenue peut être utilisée directement, par exemple pour époxyder du propylène. On recueille ainsi une solution d'oxyde de propylène dans le 1,2-dichloropropane contenant en outre de l'acide propionique et du propylène qui n'a pas réagi. Une première distillation de cette solution permet de receuillir en tête de colonne, le propylène qui n'a pas réagi. Une seconde distillation donne, en tête de colonne, l'oxyde de propylène et, en pied, une solution d'acide propionique dans le 1,2-dichloropropane. Cette solution peut être utilisée directement pour effectuer la réaction de fabrication de l'acide perpropionique.

**Revendications**

1. Procédé pour la fabrication de peracides carboxyliques en continu par réaction de l'acide carboxylique correspondant avec le peroxyde d'hydrogène en présence d'un catalyseur et d'un liquide organique inerte solvant du peracide et capable de former avec l'eau un azéotrope hétérogène, dans lequel on élimine de l'eau présente dans le mélange réactionnel par distillation de l'azéotrope eau-liquide organique, caractérisé en ce que l'on maintient dans le mélange réactionnel une quantité d'eau suffisante pour qu'il se forme une phase aqueuse distincte d'une phase organique qui contient de l'acide carboxylique, du peracide carboxylique et le liquide organique et en ce que l'on prélève une partie du mélange réactionnel et l'on sépare la phase aqueuse de la phase organique.

2. Procédé suivant la revendication 1, caractérisé en ce que le rapport pondéral de la phase aqueuse par rapport à la phase organique dans le mélange réactionnel est supérieur à 0,1 et inférieur à 10.

3. Procédé suivant la revendication 1 ou 2, caractérisé en ce que l'on sépare la phase aqueuse de la phase organique par décantation.

4. Procédé suivant la revendication 3, caractérisé en ce que la phase aqueuse séparée est réintroduite dans le mélange réactionnel.

5. Procédé suivant l'une quelconque des revendications 1 à 4, caractérisé en ce que l'on choisit un liquide organique tel que la solubilité de l'eau dans la phase organique est inférieure à la teneur en eau de l'azéotrope eau-liquide organique.

6. Procédé suivant l'une quelconque des revendications 1 à 5, caractérisé en ce que la quantité de catalyseur présente dans le mélange réactionnel est comprise entre 0,1 et 30 fois le poids total d'acide et de peracide carboxyliques.

7. Procédé suivant l'une quelconque des revendications 1 à 6, caractérisé en ce que le liquide organique est choisi parmi les esters carboxyliques, les éthers, les hydrocarbures halogénés, les hydrocarbures non substitués, les hydrocarbures substitués par des groupes nitro et les esters non acides d'acides nitrique, phosphorique et carbonique.

8. Procédé suivant l'une quelconque des revendications 1 à 7, caractérisé en ce que l'acide carboxylique est l'acide propionique ou l'acide acétique.

9. Procédé suivant l'une quelconque des revendications 1 à 8, caractérisé en ce que le catalyseur est choisi parmi l'acide sulfurique, les acides alkyl-, aryl-, arylalkyl- et alkylaryl-sulfoniques, l'acide phosphorique, les phosphates acides d'alkyle, d'aryle, d'alkylaryle et d'arylalkyle, l'acide trifuoracétique, l'acide acétylsulfoacétique et les résines échangeuses d'ions sulfonées.

10. Procédé suivant l'une quelconque des revendications 1 à 9, caractérisé en ce que le liquide organique est choisi parmi le 1,2-dichloropropane et le 1,2-dichloréthane, l'acide carboxylique est l'acide propionique, et le catalyseur est l'acide sulfurique.

11. Utilisation de la phase organique contenant du peracide carboxylique obtenue dans le procédé suivant l'une quelconque des revendications 1 à 10 pour la fabrication d'époxydes à partir d'oléfines, caractérisée en ce que l'on sépare successivement du mélange réactionnel provenant de la fabrication d'époxydes l'oléfine qui n'a pas réagi et l'oxyde d'oléfine produit, et en ce que l'on renvoie directement à la fabrication du peracide l'acide carboxylique sous la forme de la solution que l'on recueille.

12. Utilisation suivant la revendication 11, caractérisée en ce que l'on choisit un liquide organique dont la température d'ébullition est supérieure à celle de l'oléfine et de l'oxyde d'oléfine, qui ne forme pas d'azéotrope avec l'oléfine et l'oxyde d'oléfine, et dont les azéotropes éventuels avec l'acide carboxylique et le peracide carboxylique ont une température d'ébullition supérieure à celle de l'oléfine et de l'oxyde d'oléfine.

**Patentansprüche**

1. Kontinuierliches Verfahren zur Herstellung von Percarbonsäuren durch Umsetzung der entsprechenden Carbonsäure mit Wasserstoffperoxid in Anwesenheit eines Katalysators und einer organischen inerten Flüssigkeit, die ein Lösungsmittel für die Persäure darstellt und in der Lage ist, mit Wasser ein heterogenes Azeotrop zu bilden, wobei man das in der Reaktionsmischung anwesende Wasser durch Destillation des Azeotrops aus Wasser und organischer Flüssigkeit entfernt, dadurch gekennzeichnet, dass man in der Reaktionsmischung eine Wassermenge beibehält, die ausreichend ist, dass sich eine wässrige Phase bildet unterschiedlich von einer organischen Phase, die die Carbonsäure, die Percarbonsäure und die organische Flüssigkeit enthält, und dass man einen Teil der Reaktionsmischung abzieht und die wässrige Phase von der organischen Phase trennt.

2. Verfahren gemäss Anspruch 1, dadurch gekennzeichnet, dass das Gewichtsverhältnis der wässrigen Phase zur organischen Phase in der Reaktionsmischung oberhalb 0,1 und unterhalb 10 liegt.

3. Verfahren gemäss Anspruch 1 oder 2, dadurch gekennzeichnet, dass man die wässrige Phase von der organischen Phase durch Dekantieren abtrennt.

4. Verfahren gemäss Anspruch 3, dadurch gekennzeichnet, dass die abgetrennte wässrige Phase wieder in die Reaktionsmischung zurückgeführt wird.

5. Verfahren gemäss einem der Ansprüche 1 bis 4, dadurch gekennzeichnet, dass man eine solche organische Flüssigkeit auswählt, dass die Löslichkeit des Wassers in der organischen Phase unterhalb des Wassergehaltes des Azeotrops aus Wasser und organischer Flüssigkeit liegt.

6. Verfahren gemäss einem der Ansprüche 1 bis 5, dadurch gekennzeichnet, dass die Menge des in der Reaktionsmischung anwesenden Katalysators zwischen dem 0,1 und 30fachen des Gesamtgewichts von Säure und Percarbonsäure liegt.

7. Verfahren gemäss einem der Ansprüche 1 bis 6, dadurch gekennzeichnet, dass die organische Flüssigkeit ausgewählt ist unter den Carbonsäureestern, den Äthern, den halogenierten Kohlenwasserstoffen, den nicht-substituierten Kohlenwasserstoffen, den mit Nitrogruppen substituierten Kohlenwasserstoffen und den nichtsauren Estern der Stickstoffsäuren, Phosphorsäuren und Carbonsäuren.

8. Verfahren gemäss einem der Ansprüche 1 bis 7, dadurch gekennzeichnet, dass die Carbonsäure Propionsäure oder Essigsäure ist.

9. Verfahren gemäss einem der Ansprüche 1 bis 8, dadurch gekennzeichnet, dass der Katalysator ausgewählt wird unter Schwefelsäure, den Alkyl-, Aryl-, Arylalkyl- und Alkyl-arylsulfonsäuren. Phosphorsäure, den sauren Alkylphosphaten, Arylphosphaten, Alkylarylphosphaten und Arylalkylphosphaten, Trifluoressigsäure, Acetylsulfoessigsäure und den sulfonierten Ionenaustauscherharzen.

10. Verfahren gemäss einem der Ansprüche 1 bis 9, dadurch gekennzeichnet, dass die organische Flüssigkeit ausgewählt ist unter 1,2-Dichlorpropan und 1,2-Dichloräthan, dass die Carbonsäure Propionsäure ist und dass der Katalysator Schwefelsäure ist.

11. Verwendung der organischen, Percarbonsäure enthaltenden Phase, erhalten nach dem Verfahren gemäss einem der Ansprüche 1 bis 10 zur Herstellung von Epoxiden, ausgehend von Olefinen, dadurch gekennzeichnet, dass man nacheinander aus dem Reaktionsgemisch der Herstellung von Epoxiden das nichtreagierte Olefin und das hergestellte Olefinoxid abtrennt und dass man die Carbonsäure in Form der erhaltenen Lösung direkt der Fabrikation der Persäure zuführt.

12. Verwendung gemäss Anspruch 11, dadurch gekennzeichnet, dass man eine organische Flüssigkeit wählt, deren Siedepunkt oberhalb desjenigen des Olefins und des Olefinoxids liegt, die kein Azeotrop mit dem Olefin und dem Olefinoxid bildet und deren Azeotrope mit der Carbonsäure und der Percarbonsäure Siedepunkte oberhalb derjenigen des Olefins und des Olefinoxids besitzen.

## Claims

1. Continuous process for the manufacture of percarboxylic acids by reacting the corresponding carboxylic acid with hydrogen peroxide in the presence of a catalyst and of an inert organic liquid which is a solvent for the peracid and is capable of forming a heterogeneous azeotrope with water, in which water present in the reaction mixture is removed by distillation of the water/organic liquid azeotrope, characterised in that water is kept in the reaction mixture in a sufficient amount for the formation of an aqueous phase which is distinct from an organic phase containing the carboxylic acid, percarboxylic acid and organic liquid and that part of the reaction mixture is removed and the aqueous phase is separated from the organic phase.

2. Process according to Claim 1, characterised in that the weight ratio of the aqueous phase to the organic phase in the reaction mixture is greater than 0,1 and less than 10.

3. Process according to Claim 1 or 2, characterised in that the aqueous phase is separated from the organic phase by decantation.

4. Process according to Claim 3, characterised in that the aqueous phase separated off is reintroduced into the reaction mixture.

5. Process according to any one of Claims 1 to 4, characterised in that the organic liquid is chosen such that the solubility of water in the organic phase is less than the water content of the water/organic liquid azeotrope.

6. Process according to any one of Claims 1 to 5, characterised in that the amount of catalyst present in the reaction mixture is between 0.1 and 30 times the total weight of carboxylic acid and percarboxcylic acid.

7. Process according to any one of Claims 1 to 6, characterised in that the organic liquid is chosen from amongst carboxylic acid esters, ethers, halogenohydrocarbons, unsubstituted hydrocarbons, hydrocarbons substituted by nitro groups, and non-acid esters of nitric, phosphoric and carbonic acids.

8. Process according to any one of Claims 1 to 7, characterised in that the carboxylic acid is propionic acid or acetic acid.

9. Process according to any one of Claims 1 to 8, characterised in that the catalyst is chosen from amongst sulphuric acid, alkyl-, aryl-, arylalkyl- and alkylaryl-sulphonic acids, phosphoric acid, alkyl, aryl, alkylaryl and arylalkyl acid phosphates, trifluoroacetic acid, acetylsulphoacetic acid and sulphonated ion exchange resins.

10. Process according to any one of Claims 1 to 9, characterised in that the organic liquid is chosen from amongst 1,2-dichloropropane and 1,2-dichloroethane, the carboxylic acid is propionic acid and the catalyst is sulphuric acid.

11. Use of the organic phase containing percarboxylic acid, obtained in the process according to any one of Claims 1 to 10, for the manufacture of epoxides from olefines, characterised in that the unreacted olefine and the olefine oxide produced are successively separated from the reaction mixture originating from the manufacture of epoxides, and in that the carboxylic acid, in the form of the solution which is collected, is returned directly to the manufacture of the peracid.

12. Use according to Claim 11, characterised in that an organic liquid is chosen, the boiling point of which is higher than that of the olefine and that of the olefine oxide, which does not form an azeotrope with the olefine and the olefine oxide, and of which the possible azeotropes with the carboxylic acid and the percarboxylic acid have a boiling point which is higher than that of the olefine and that of the olefine oxide.

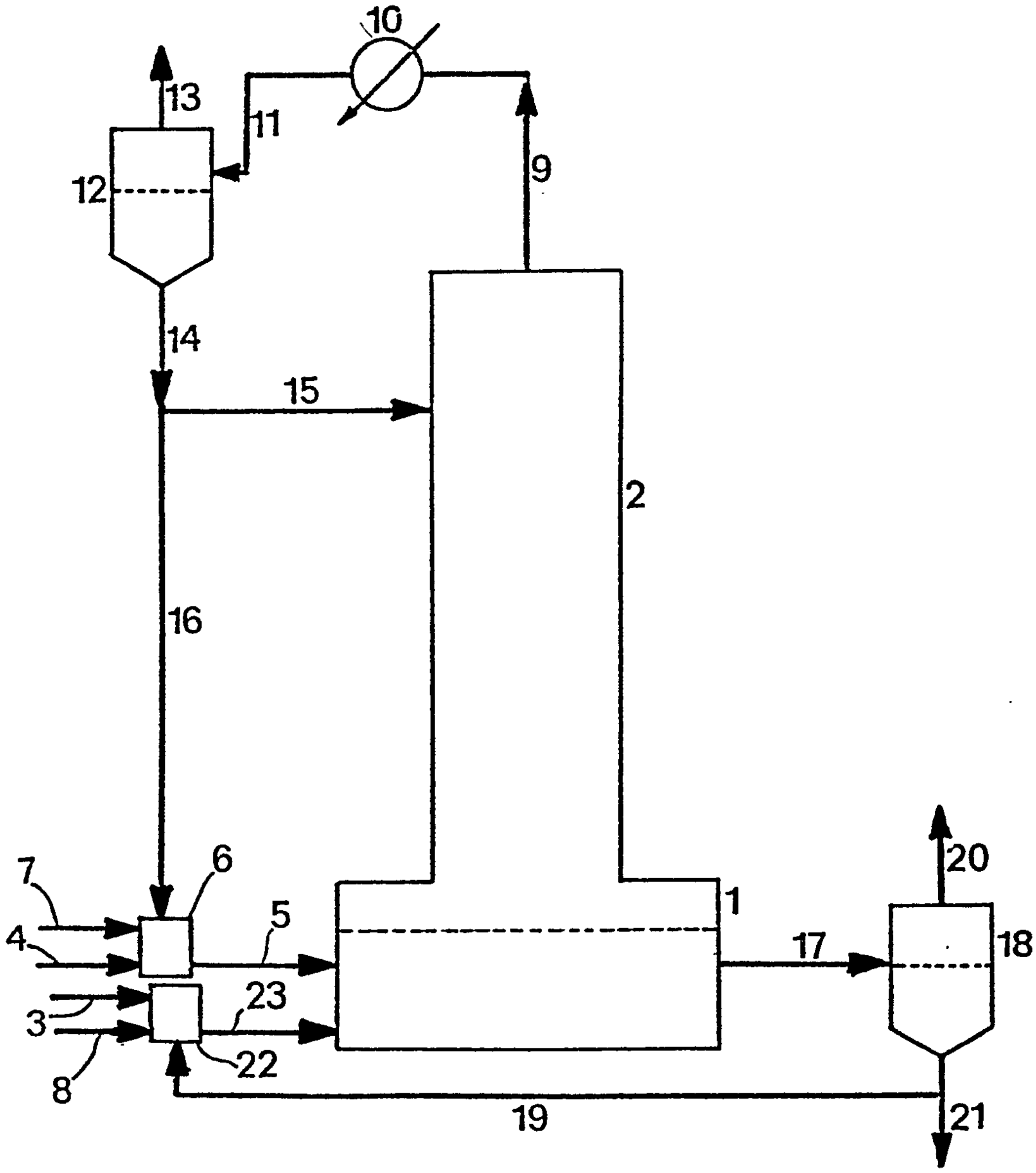
10
13
11
12
9
14
15
2
16
20
7
6
1
4
5
17
18
3
23
22
8
19
21